(19) Europäisches Patentamt
European Patent Office
Office européen des brevets

(11) Publication number: **0 037 175**
**B1**

(12) **EUROPEAN PATENT SPECIFICATION**

(45) Date of publication of patent specification: **10.02.88**

(21) Application number: **81300867.9**

(22) Date of filing: **03.03.81**

(51) Int. Cl.[4]: **A 61 K 31/425**, A 61 K 31/43, A 61 K 31/44, A 61 K 31/47, A 61 K 31/49, A 61 K 31/475, A 61 K 31/485, A 61 K 31/585, A 61 K 33/30, A 61 K 45/02, A 61 K 45/06

(54) **Pharmaceutical compositions.**

(30) Priority: **14.03.80 GB 8008764**

(43) Date of publication of application:
**07.10.81 Bulletin 81/40**

(45) Publication of the grant of the patent:
**10.02.88 Bulletin 88/06**

(84) Designated Contracting States:
**AT BE CH DE FR GB IT LI LU NL SE**

(58) References cited:
**EP-A-0 003 407**
**EP-A-0 004 770**
**EP-A-0 019 423**
**BE-A- 867 787**
**FR-A-2 444 459**
**FR-M- 1 592**
**FR-M- 3 184**
**US-A-3 993 775**

**L F and M Fieser, Organic Chemistry, 3rd ed. 1956, page 401**

(73) Proprietor: **EFAMOL LIMITED**
**71/74 Mark Lane**
**London E.C.3 (GB)**

(72) Inventor: **Horrobin, David Frederick**
**P.O. Box 10 Nuns Island**
**Montreal H3E 1J8 (CA)**

(74) Representative: **Caro, William Egerton et al**
**J. MILLER & CO. Lincoln House**
**296-302 High Holborn**
**London WC1V 7JH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 037 175 B1

Courier Press, Leamington Spa, England.

**Description**

Field of the Invention

This invention relates to compositions for the treatment of cancer primarily, but not exclusively, in the field of human medicine.

Transformation

The drug thioproline (thiazolidine-4-carboxylic acid), of formula

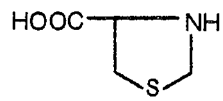

has recently been found to have an anti-cancer effect in humans (Brugarolas et al. Lancet, page 68, 12th January 1980). The effect of the drug was discovered because of its ability to induce what is known as reverse transformation in certain cultured cell lines.

These cell lines, derived from normal human and animal cells, behave in many respects like spontaneously occurring cancer cells. Normal cells can be transformed by agents such as radiation, injection with certain viruses and by chemical carcinogens. The transformed cells multiply rapidly, like cancer cells, and generally show the morphological and biochemical characteristics of such cells, as discussed further for example in Johnson et al, Proc. Nat. Acad. Sc. U.S.A., *68* 425—429 (1975) and Puck, ibid, *74* 4491—4495 (1977).

Relation to Essential Fatty Acid Metabolism

One particular characteristic shown by human and animal cancer cells and by transformed cells is a consistent absence of the enzyme delta-6-desaturase which converts linoleic acid to γ-linolenic acid. The inventor believes that this fact is of great significance and that faulty essential fatty acid metabolism is a key factor in cancer.

The pathways of EFA transformation in the body are in outline as below:

cis—linoleic acid
(9,12—octadecadienoic acid)
↓
γ—linolenic acid (GLA)
(6,9,12—octadecatrienoic acid)
↓
DGLA ester reserves (small) ⇌ dihomo—γ—linolenic acid (DGLA) → 1 series PG'S
↓
AA

Large AA ester reserves ⇌ Arachidonic acid (AA)
(5,8,11,14—eicosatetraenoic acid) → 2 series PG's

The broad outline of the pathways is well known, and it brings out clearly that a major function of essential fatty acids (EFAs) is to act as precursors for prostaglandins, 1-series PGs being formed from dihomo-γ-linolenic acid (DGLA) and 2-series PGs from arachidonic acid (AA). DGLA and AA are present in food in only small quantities, and the major EFA in food is linoleic acid which is first converted to γ-linolenic acid (GLA) and then to DGLA and AA. The conversion of linoleic acid to GLA is blocked by a high fat and high carbohydrate diet, by ageing and for emxamle by diabetes. Stores of AA in the body in the form of lipid esters are very large indeed. In contrast only small amounts of DGLA ester are present.

DGLA is the key substance. GLA is almost completely and very rapidly converted in the body to DGLA and so for practical purposes the oral administration of DGLA and GLA amount to the same thing. DGLA

can be converted to a storage form, or to PGs of the 1-series, or to arachidonic acid and thence to PGs of the 2-series. The conversion to arachidonic acid is irreversible.

Accordingly it can be seen that since γ-linolenic acid is a necessary precursor of dihomo-γ-linolenic acid and thus of 1-series PGs, and since also cellular stores of DGLA are very limited, cancer cells and transformed cells soon lose the ability to make 1-series PGs and in particular the important compound PGE 1.

Significance

The inventor believes that many of the characteristic features of transformed and cancer cells are due to this damage to PG metabolism and accordingly further than thioproline will be more effective in the treatment of cancer, particularly in the long term, if supported by measures to restore production of 1-series PGs and particularly PGE 1 in the cells. Such measures lie in particular in the provision of GLA or DGLA from either natural or synthetic sources, to by-pass the block at the delta-6-desaturase and enable cells which had lost the enzyme to continue to make PGE 1.

It may be noted that having regard to the inventor's view of the connection between 1-series PG metabolism and reverse transformation, it would be expected that substances such as PGE 1 might themselves induce the reverse transformation process. It is therefore significant that it is in fact known that PGE 1 is able to induce reverse transformation in cultured cells, possibly through stimulating production of a nucleotide known as cyclic AMP which is also known to induce the transformation. These reverse transformation inducing properties have been known since 1971, but the inventor believes that he is the first to recognise their significance in the present context through his concern with prostaglandin metabolism generally, shown for example in his previous patent applications referred to below. Through his approach, it has been possible to see that, while substances such as PGE 1 and cyclic AMP would never be thought of as possible components of therapeutic compositions, being unstable and generally unsuitable for administration, restoration of the natural in situ production of 1-series PGs and in particular PGE 1 is of great value. This value has been shown directly in the female Fisher rat where the growth in both size and weight of the transplantable R323OAC mammary tumour is halved by 25 μl Evening Primrose oil daily, as a GLA source directly favouring 1-series PG production. Moreover direct evidence of effectiveness of administration of GLA in the form of Oenothèra seed oil, combined with Vitamin C, has already been obtained from early results in a group of cancer patients in a Scottish hospital. One man with a papillary bladder carcinoma of fifteen years standing and beyond help by surgery, radiotherapy or conventional chemotherapy has had haematuria controlled over more than a year, with accompanying well being rather than the well known toxic effects of conventional treatments. Similarly, a woman with the whole abdomen infiltrated with an anaplastic carcinoma extensively palpable and confirmed by histological examination, untreatable by normal means, has shown complete regression of the tumours to palpation over a period of fifteen months, again with accompanying well being. Such results indicate the value of the combined approach of such measures with the present invention discussed below.

The Invention

The invention accordingly lies in a pharmaceutical composition comprising trioproline in combination with γ-linolenic acid and/or dihomo-γ-linolenic acid, optionally in association with linoleic and if desired other fat acids, said γ-linolenic or dihomo-γ-linolenic acids being used if desired as physioligically functional salt, ester or other derivatives thereof.

Relationship to Previous Proposals

The above approach may be used in combination with the use of other materials as disclosed in the inventor's EP—A—003407, EP—A—0004770 and EP—A—0019423.

These materials include zinc, penicillin and β-lactam antibiotics generally, (EP—A—0003407) and also penicillamine, phenformin and levamisole (EP—A—0004770) when the other effects of these materials are acceptable, all of which are believed to enhance mobilisation of DGLA reserves and hence ensure that administered GLA and DGLA go into synthesis of PGs. The materials also include ascorbic acid, nalorphine, levallorphan (published European Patent Application No. 0079423 etc.), a class which enhance physiological synthesis of 1-series PGs from DGLA without substantially enhancing synthesis of 2-series PGs from AA.

Further, there is evidence that thromboxane A2 (produced in the body along with the endoperoxides giving rise to 2-series PGs) indirectly enhances formation of PGE 1. Substances such as colchicine, amantadine, griseofulvin; vinblastine, interferon and melatonin, which are also discussed in the pending patent applications (published European Patent Application No. 0004770 etc.) and which seem to increase production or action of thromboxane A2, are thus also desirably used in the composition of the present invention.

Reference may be made to the above published specifications for further details. The materials of the present invention may also be used in conjunction with chloroquine, amodiaquine and hydroxychloroquine; diidohydroxyquin iodochlorhydroxyquin, chiniofon, pentaquine, isopentaquine and primaquine; quinacrine (mepacrine); quinidine, quinine and procaine; emetine, metronidazole; and spironolactone all of which influence the 1-series/2-series PG balance in the body in favour of 1-series PGs.

The purpose of these materials is thus not only to help in the restoration of 1-series PG production but to maintain a proper 1-series/2-series PG balance.

Other published material raised in the search report and by the Examiner, none of it seen as leading to the invention, is:

U.S. 3 993 775 Williams (filed 1974). Immuno-suppression in control of multiple sclerosis and other disorders, by use of GLA or DGLA.

Belgian 867 787 Kali-Chemie (filed 1978). Use of $C_6$—$C_{12}$ fatty acids, optionally with longer chain acids such as oleic acid, to encourage resorption of medicaments.

French Medicament Patent 3184M Sogespar (filed 1963) Thiazolidine carboxylic acid in liver disturbances.

French Medicamnt Patent 1592M Aschaffenburger (filed 1961) Thiazolidine carboxylic acid for the hair and against dandruff.

French 2 444 459 Gosalvez (filed 1979) Thiazolidine carboxylic acid against cancer.

"The Pharmacological Basis of Therapeutics" MacMillan, New York (1967) Ch. 62, p. 1374. Actions of vinca alkaloids.

Effective Agents

Thus DGLA or GLA from any natural or synthetic source alone or with one or more of the agents discussed above is proposed for use with thioproline to restore 1-series PG production.

Convenient physioligically functional derivatives of γ-linolenic acid and dihomo-γ-linolenic acid for use according to the invention include the $C_1$—$C_4$ alkyl (e.g. methyl and ethyl) esters and the glycerides of the acids.

If desired, pharmaceutical compositions may be produced for use in the invention by associating natural or synthetic γ-linolenic acid (or a physiologically functional derivative thereof) and/or dihomo-γ-linolenic acid (or a physiologically functional derivative thereof), as such, with an acceptable pharmaceutical vehicle. It is at present convenient to incorporate the γ-linolenic acid into compositions in the form of an available oil having a high γ-linolenic acid content, hence references to "oil" herein.

At the present time known natural sources of oils having a high γ-linolenic acid content are few (there are no known natural sources of significant amounts of dihomo-γ-linolenic acid). One source of oils currently available is the seed of Evening Primrose species such as *Oenothera biennis L.* and *Oenothera lamarckiana*, the oil extract therefrom containing γ-linolenic acid (about 8%) and linoleic acid (about 72%) in the form of their glycerides together with other glycerides (percentages based on total fatty acids). Another source of γ-linolenic acid is Borage species such as *Borago officinalis* which, though its current yield per acre is low, provides a richer source of γ-linolenic acid than Oenothera oil. Recent studies on fungi which can be cultivated by fermentation promise a fungal oil source.

The seed oil extracts referred to above can be used as such or can for example if desired be fractionated to yield an oily composition containing the triglyucerides of γ-linolenic and linoleic as the main fatty acid components, the γ-linolenic acid content being if desired a major proportion. Seed oil extracts appear to have a stabilising effect upon any dihomo-γ-linolenic-acid or physiologically functional derivative thereof incorporated therein.

Amounts of γ-linolenic Acid and Related Materials

A preferred daily dosage for all purposes for an adult (weight ca 75 kg) is from 0.05 to 0.1 up to 1, 2, 5 or even 10 g as required of γ-linolenic acid or equivalent weight (calculated as γ-linolenic acid) or a physiologically functional derivative thereof. Amounts may in particular be 0.1 to 1.0 g daily. Such doses correspond to about 2 to 20 g daily of the Oenothera oil discussed below. In place of, or in addition to, γ-linolenic acid, one may use dihomo-γ-linolenic acid or a physiologically functional derivative thereof in amount equivalent in molar terms to γ-linolenic acid and calculated as such. This dosage can for example be taken as a single dose or divides into 2, 3 or 4 subdivisions thereof as convenient.

Amount of Thioproline

Amounts may be for example 100 mg to 35 g/day, conveniently 40 mg/kilo body weight/day (ca 3 g/day).

Amount of other Active Materials

Amounts of materials used as referred to above to augment the effect of the GLA and/or DGLA may for example be:

| | | |
|---|---|---|
| I | Assimilable zinc compound | 2.5 to 800 mg/day, preferably 10—80 mg, calculated as zinc |
| | Penicillin V or other β-lactam antibiotics | 0.5 to 10 g/day |
| | Penicillamine | 50 mg to 10 g/day |
| | Phenformin | 10 mg to 5 g/day |
| | Levamisole | 10 mg to 2 g/day |

| | | |
|---|---|---|
| II | Colchicine | 0.3 to 15 mg/day, preferably 0.6 to 2.4 mg |
| | Amantadine | 100 to 1000 mg/day |
| | Griseofulvin | 0.5 to 5 g/day |
| | Vinblastine | 35 to 350 mg/week |
| | Interferon | $1 \times 10^5$ to $1 \times 10^8$ units/day |
| | Melatonin | 10 mg to 5 g/day |
| III | Ascorbic acid | 50 mg to 50 g/day |
| | Naloxone | 0.1 to 500 mg/day |
| | Nalorphine | 1 mg to 5 g/day |
| | Levallorphan | 0.2 mg to 1 g/day |
| IV | Chloroquine and other quinolines listed with it earlier, | 250 mg/week to 2500 mg/day |
| | Quinacrine | 100 to 2500 mg/day |
| | Quinine | 100 mg to 10 g/day |
| | Quinidine | 100 mg to 2 g/day |
| | Procaine | 100 mg to 10 g/day |
| | Spironolactone | 30 mg to 2 g/day |
| | Emetine | 10 to 100 mg/day |
| | Metronidazole | 100 mg to 10 g/day |

Packs

If it is not desired to have compositions comprising active materials listed above, packs may be prepared comprising the materials presented for separate or part joint and part separate administration in the appropriate relative amounts, and such packs are within the purview of the invention.

Dietary Compositions

The invention is chiefly described in terms of pharmaceutical compositions, but it will be uderstood that the γ-linolenic and other acids, being in the nature of dietary supplements, could be incorporated in a dietary margarine or other foodstuffs; such foodstuffs, possibly containing other active materials and generally referred to in this description as dietary or pharmaceutical compositions, are within the purview of the invention and thus of the term pharmaceutical compositions, packs or the like used herein.

Veterinary Applications

It will be understood that where a disorder of a kind calling for treatment in animals arises, the invention while described primarily in terms of human medicine and treatment is equally applicable in the veterinary field.

Pharmaceutical Presentation

The compositions according to the invention are conveniently in a form suitable for oral, rectal, parenteral or topical administration in a suitable pharamceutical vehicle, as discussed in detail for example in U.K. Patent Specification No. 1 082 624 and in any case very well known generally for any particular kind of preparation. Thus for example tablets, capsules, ingestible liquid or powder preparations, creams and lotions for topical application, or suppositories, can be prepared as required. Injectable solutions of hydrolysed Oenothera oil may be prepared using albumin to solubilise the free acid.

Advantageously a preservative is incorporated into the preparations. α-Tocopheral in a concentration of about 0.1% by weight has been found suitable for the purpose.

It will be understood that the absolute quantity of active ingredients present in any dosage unit should not exceed that appropriate to the rate and manner of administration to be employed but on the other hand should also desirably be adequate to allow the desired rate of administration to be achieved by a small number of doses. The rate of administration will moreover depend on the precise pharmacological action desired.

The following Examples serve to illustrate pharmaceutical compositions useful in treatment according to the invention:

## Examples

Pharmaceutical compositions containing a unit dose of an oil extract from the seeds of *Oenothera biennis L.* optionally with methyl dihomo-γ-linolenate and/or zinc oleate, penicillin V, colchicine or any of the other active materials referred to herein, are prepared by encapsulation of the natural oil in soft gelatin capsules manufactured by known methods.

The oil is extracted from the seeds by one of the conventional methods of extraction such as cold pressure, screw pressure after partially cooking the seed, or solvent extraction.

Fractionation of a typical sample of this oil shows a yield of 97.0% oil in the form of methyl esters, with the relative proportions:

| | |
|---|---|
| Palmitate | 6.15 |
| Stearate | 1.6 |
| Oleate | 10.15 |
| Linoleate | 72.6 |
| α-linolenate | 8.9 |

As preservative, α-tocopherol is added to the oil in a concentration of 0.1%.

Gelatin capsules containing oil extracts prepared as described above, each having the following contents of active ingredients (0.5 g oil extact = ca 0.045 g γ-linolenic acid), are prepared in conventional fashion.

## Example 1

The following capsules may be given, two capsules three times a day, in the treatment of cancer:

Capsules containing:

| | |
|---|---|
| Oenothra Oil extract (above) | 0.5 g |
| Thioproline | 500 mg |

## Further Examples A

Similarly capsules containing additional materials may be administered, for example, 10 mg methyl dihomo-γ-linolenate per capsule as a direct supplement to the oil; or for example for their indirect action zinc oleate 10 mg or penicillin V 0.25 g (compare the Examples of published European PaAtent Application No. 0003407); or phenformin 25 mg, levamisole 25 mg or penicillamine 100 mg per capsule, or colchicine 0.2 mg, amantadine 100 mg or griseofulvin 0.5 mg per capsule (compare the Examples of published European Patent Application No. 0004770 and also the use in conjunction with vinblastine, melatonin and interferon referred to therein); or ascorbic acid 100 mg, naloxone 5 mg, nalorphine 5 mg or levallorphan 5 mg (compare the Examples of published European Patent Application No. 0019423).

## Further Examples B

Other materials that may be incorporated in capsules as in Example 1 are for example chloroquine 50 mg, spironolactone 50 mg, quinacrine 50 mg, quinine or quinidine 50 mg, emetine 50 mg, or procaine 100 mg.

It will be understood throughout that while a full theoretical discussion of what is believed to be the reason for the effectiveness of the compositions proposed is given to aid understanding, the invention is in no way to be limited by this discussion.

**Claims for the Contracting States: CH DE FR GB IT LI LU NL SE BE**

1. A pharmaceutical composition comprising γ-linolenic acid and/or dihomo-γ-linolenic acid or a physiologically functional salt, ester or other derivative thereof, and thioproline, alone or in an acceptable pharmaceutical vehicle.

2. A composition according to claim 1, further comprising a physiologically assimilable zinc compound, a β-lactam antibiotic, penicillamine, phenformin or levamisole.

3. A composition according to claim 1, further comprising colchicine; or vinblastine; or griseofulvin, amantadine or melatonin; or interferon.

4. A composition according to claim 1, further comprising Vitamin C; or naloxone, nalorphine or levallorphan.

5. A composition according to claim 1, further comprising a quinoline derivative selected from chloroquine, amodiaquine, hydroxychloroquine, diiodohydroxyquin, iodochlorhydroxyquin, chiniofon, pentaquine, isopentaquine or primaquine; quinacrine (mepacrine), quinidine, quinine, or procaine; emetine or metronidazole; or spironolactone.

6. A composition according to any preceding claim, presented for administration in quantities to give 0.05 to 10 g/day of said γ-linolenic or dihomo-γ-linolenic acid or derivative, calculated as γ-linolenic acid.

7. A composition according to any preceding claim, presented for administration in quantities to give 100 mg to 35 g/day thioproline.

8. A composition according to claim 2, presented for administration in quantities to give 2.5 to 800 mg/day assimilable zinc compound calculated as zinc, 0.05 to 10 g/day β-lactam antibiotic, 50 mg to 10 g/day penicillamine, 10 mg to 6 g/day phenformin or 10 mg to 2 g/day levamisole.

9. A composition according to claim 3, presented for administration in quantities to give doses of:
0.3 to 15 mg/day colchicine
100 to 1000 mg/day amantadine
0.5 to 5 g/day griseofulvin
35 to 350 mg/week vinblastine
$1 \times 10^5$ to $1 \times 10^8$ units/day interferon, or
10 mg to 5 g/day melatonin.

10. A composition according to claim 4, presented for administration in quantities to give doses of:
50 mg to 50 g/day ascorbic acid or
0.1 to 500 mg/day naloxone, 1 mg to 5 g/day nalorphine, or 0.2 mg to 1 g/day levallorphan.

11. A composition according to claim 5, presented for administration in quantities to give doses of 250 mg/week to 2500 mg/day of the quinoline derivative; 100 to 2500 mg/day of quinacrine; 100 mg to 10 g/day of quinine, 100 mg to 2 g/day of quinidine, or 100 mg to 10 g/day of procaine; 10 to 100 mg/day emetine; or 100 mg to 10 g/day metronidazole or 30 mg to 2 g/day of spironolactone.

12. A composition according to any preceding claim, wherein the γ-linolenic acid is present in the form of the oil of the seed of Oenothera biennis, O. Lamarckiana or other Evening Primrose species, or a fraction thereof.

13. A composition according to any of claims 1 to 11, wherein the γ-linolenic acid is present in the form of the oil of the seed of Borago officinalis or other Borage species, or a fraction thereof.

14. The use of γ-linolenic acid and/or dihomo-γ-linolenic acid or a physiologically functional salt, ester or other derivative thereof and of thioproline in the manufacture of a product for the conjoint treatment of cancer.

15. A pharmaceutical pack comprising the materials set out in any preceding claim including at least the two essential components set out in claim 1 presented separately, or one or more separately and others together, but for conjoint administration.

**Claims for the Contracting State: AT**

1. The use of γ-linolenic acid and/or dihomo-γ-linolenic acid or a physiologically functional salt, ester or other derivative thereof, together with thioproline, alone or in an acceptable pharmaceutical vehicle for the method of producing a pharmaceutical composition for treatment of cancer.

2. the use set out in claim 1, the composition further comprising a physiologically assimilable zinc compound, a β-lactam antibiotic, penicillamine, phenformin or levamisole.

3. The use set out in claim 1, the composition further comprising colchicine; or vinblastine; or griseofulvin, amantadine or melatonin; or interferon.

4. The use set out in claim 1, the composition further comprising Vitamin C; or naloxone, nalorphine or levallorphan.

5. The use set out in claim 1, the composition further comprising a quinoline derivative selected from chloroquine, amodiaquine, hydroxychloroquine, diiodohydroxyquine, iodochlorhydroxyquin, chiniofon, pentaquine, isopentaquine or primaquine, quinacrine (mepacrine), quinidine, quinine, or procaine; emetine or metronidazole; or spironolactone.

6. The use set out in any preceding claim, the composition being presented for administration in

quantities to give 0.05 to 10 g/day of said γ-linolenic or dihomo-γ-linolenic acid or derivative, calculated as γ-linolenic acid.

7. The use set out in any preceding claim, the composition being presented for administration in quantities to give 100 mg to 35 g/day thioproline.

8. The use set out in claim 2, the composition being presented for administration in quantities to give 2.5 to 800 mg/day assimilable zinc compound calculated as zinc, 0.05 to 10 g/day β-lactam antibiotic, 50 mg to 10 g/day penicillamine, 10 mg to 5 g/day phenformin or 10 mg to 2 g/day levamisole.

9. The use set out in claim 3, the composition being presented for administration in quantities to give doses of:

0.3 to 15 mg/day colchicine
100 to 1000 mg/day amantadine
0.5 to 5 g/day griseofulvin
35 to 350 mg/week vinblastine
$1 \times 10^5$ to $1 \times 10^8$ units/day interferon, or
10 mg to 5 g/day melatonin.

10. The use set out in claim 4, the composition being presented for administration in quantities to give doses of:

50 mg to 50 g/day ascorbic acid or
0.1 to 500 mg/day naloxone, 1 mg to 5 g/day nalorphine, 0.2 mg to 1 g/day levallorphan.

11. The use set out in claim 5, the composition being presented for administration in quantities to give doses of 250 mg/week to 2500 mg/day of quinoline derivative; 100 to 2500 mg/day of quinacrine; 100 mg to 10 g/day of quinine, 100 mg to 2 g/day of quinidine, or 100 mg to 10 g/day of procaine; 10 to 100 mg/day emetine; or 100 mg to 10 g/day metronidazole; or 30 mg to 2 g/day of spironolactone.

12. The use set out in any preceding claim the γ-linolenic acid being present in the form of the oil of the seed of Oenothera biennis, O. Lamarckiana or other Evening Primrose species, or a fraction thereof.

**Patentansprüche für die Vertragsstaaten: CH DE FR GB IT LI LU NL SE BE**

1. Pharmazeutische Zusammensetzung enthaltend γ-Linolensäure und/oder Dihomo-γ-Linolensäure oder ein physioligisch wirksames Salz, einen Ester oder ein anderes Derivat derselben und Thioprolin, allein oder in einem geeigneten pharmazeutischen Träger.

2. Zusammensetzung nach Anspruch 1, zusätzlich enthaltend eine physiologisch verwertbare Zinkverbindung, ein β-Lactamintibiotikum, Penicillamin, Phenformin oder Levamisol (?).

3. Zusammensetzung nach Anspruch 1, zusätzlich enthaltend Colchicin oder Vinblastin oder Griseofulvin, Amantadin oder Melatonin oder Interferon.

4. Zusammensetzung nach Anspruch 1, zusätzlich enthaltend Vitamin C oder Naloxon, Nalorphin oder Levallorphan.

5. Zusammensetzung nach Anspruch 1, zusätzlich enthaltend ein Chinolinderivat, ausgewählt aus der Gruppe Chloroquin, Amodiaquin, Hydroxychloroquin, Diiodohydroxyquin, lodochlorhydroxyquin, Chiniofonum, Pentaquin, Isopentaquin oder Primaquin; Chinacrin (Mepacrin), Chinidin, Chinin oder Procain; Emetin oder Metronidazol; oder Spirinolacton.

6. Zusammensetzung nach einem der vorstehenden Ansprüche, vorliegend zur Anwendung in Mengen, die 0,05 bis 10 g/Tag γ-Linolensäure oder Dihomo-γ-Linolensäure oder deren Derivate, berechnet als γ-Linolensäure, ergeben.

7. Zusammensetzung nach einem der vorstehenden Ansprüche, vorliegend zur Anwendung in Mengen, die 100 mg bis 35 g/Tag Thioprolin ergeben.

8. Zusammensetzung nach Anspruch 2, vorliegend zur Anwendung in Mengen, die 2,5 bis 800 mg/Tag verwertbare Zinkverbindung ergeben, berechnet als Zink, 0,05 bis 10 g Tag β-Lactamantibiotikum, 50 mg bis 10 g/Tag Penicillamin, 10 mg bis 5 g/Tag Phenformin oder 10 mg bis 2 g/Tag Levamisol (?).

9. Zusammensetzung nach Anspruch 3, vorliegend zur Anwendung in Mengen, die folgende Dosen ergeben:

0,3 bis 15 mg/Tag Colchicin
100 bis 1000 mg/Tag Amantadin
0,5 bis 5 g/Tag Griseofulvin
35 bis 350 mg/Woche Vinblastin
$1 \times 10^5$ bis $1 \times 10^8$ Einheiten/Tag Interferon oder 10 mg bis 5 g/Tag Melatonin.

10. Zusammensetzung nach Anspruch 4, vorliegend zur Anwendung in Mengen, die folgende Dosen ergeben:

50 mg bis 50 g/Tag Ascorbinsäure oder
0,1 bis 500 mg/Tag Naloxon,
1 mg bis 5 g/Tag Nalorphin, oder
0,2 mg bis 1 g/Tag Levallorphan.

11. Zusammensetzung nach Anspruch 5, vorliegend zur Anwendung in Mengen, die folgende Dosen ergeben:
250 mg/Woche bis 2500 mg/Tag Chinolin-Derivat
100 bis 2500 mg/Tag Chinacrin
100 mg bis 10 g/Tag Chinin
100 mg bis 2 g/Tag Chinidin oder
100 mg bis 10 g/Tag Procain
10 bis 100 mg/Tag Emetin oder
100 mg bis 10 g/Tag Metronidazol oder
30 mg bis 2 g/Tag Spironolacton.

12. Zusammensetzung nach einem der vorhergehenden Ansprüche, bei der die γ-Linolensäure vorliegt als Öl des Samens von Oenothera biennis, O. Lamarckiana oder einer anderen Nachtkerzenart oder eines Bestandteils desselben.

13. Zusammensetzung nach einem der Ansprüche 1—11, bei der die γ-Linolensäure vorliegt als Öl des Samens von Borago officinalis oder einer anderen Borago — Art oder eines Bestandteils davon.

14. Verwendung von γ-Linolensäure und/oder Dihomo-γ-Linolensäure oder eines physioligisch wirksamen Salzes, Esters oder eines anderen Derivates derselben und von Thioprolin zur Herstellung eines Produkts zur gemeinsamen Behandlung von Krebs.

15. Eine pharmazeutische Darreichungsform enthaltend die Stoffe gemäß einem der vorhergehenden Ansprüche, einschließlich wenigstens der beiden wesentlichen Komponenten gemäß Anspruch 1 allein oder eine oder mehrere getrennt und andere zusammen, aber für eine gemeinsame Verabreichung.

**Patentansprüche für den Vertragsstaat: AT**

1. Verwendung von γ-Linolensäure und/oder Dihomo-γ-Linolensäure oder einem physiologisch wirksamen Salz, Ester oder einem anderen Derivat derselben, zusammen mit Thioprolin, allein oder in einem geeigneten pharmazeutischen Träger für das Herstellungsverfahren einer pharmazeutischen Zusammensetzung zur Behandlung von Krebs.

2. Verwendung nach Anspruch 1, wobei die Zusammensetzung außerdem eine physiologisch verwertbare Zinkverbindung, ein β-Lactamintibiotikum, Penicillamin, Phenformin oder Levamisol (?) enthält.

3. Verwendung nach Anspruch 1, wobei die Zusammensetzung außerdem Colchicin oder Vinblastin oder Griseofulvin, Amantadin oder Melatonin oder Interferon enthält.

4. Verwendung nach Anspruch 1, wobei die Zusammensetzung außerdem Vitamin C oder Naloxon, Nalorphin oder Levallorphan enthält.

5. Verwendung nach Anspruch 1, wobei die Zusammensetzung außerdem ein Chinolinderivat ausgewählt aus der Gruppe Chloroquin, Amodiaquin, Hydroxychloroquin, Diiodohydroxyquin, Iodochlorhydroxyquin, Chiniofonum, Pentaquin, Isopentaquin oder Primaquin; Chinacrin (Mepacrin), Chinidin, Chinin oder Procain; Emetin oder Metronidazol; oder Spirinolacton enthält.

6. Verwendung nach einem der vorstehenden Ansprüche, wobei die Zusammensetzung vorliegt zur Anwendung in Mengen, die 0,05 bis 10 g/Tag der genannten γ-Linolensäure oder Dihomo-γ-Linolensäure oder deren Derivate, berechnet als γ-Linolensäure, ergeben.

7. Verwendung nach einem der vorstehenden Ansprüche, wobei die Zusammensetzung zur Anwendung in Mengen vorliegt, die 100 mg bis 35 g/Tag Thioprolin ergeben.

8. Verwendung nach Anspruch 2, wobei die Zusammensetzung zur Anwendung zur Anwendung in Mengen vorliegt, die 2,5 bis 800 mg/Tag verwertbare Zinkverbindung ergeben, berechnet als Zink, 0,05 bis 10 g Tag β-Lactamantibiotikum, 50 mg bis 10 g/Tag Penicillamin, 10 mg bis 5 g/Tag Phenformin oder 10 mg bis 2 g/Tag Levamisol (?).

9. Verwendung nach Anspruch 3, wobei die Zusammensetzung zur Anwendung in Mengen vorliegt, die folgende Dosen ergeben:
0,3 bis 15 mg/Tag Colchicin
100 bis 1000 mg/Tag Amantadin
0,5 bis 5 g/Tag Griseofulvin
35 bis 350 mg/Woche Vinblastin
$1 \times 10^6$ bis $1 \times 10^8$ Einheiten/Tag Interferon oder
10 mg bis 5 g/Tag Melatonin.

10. Verwendung nach Anspruch 4, wobei die Zusammensetzung zur Anwendung in Mengen vorliegt, die folgende Dosen ergeben:
50 mg bis 50 g/Tag Ascorbinsäure oder
0,1 bis 500 mg/Tag Naloxon,
1 mg bis 5 g/Tag Nalorphin, oder
0,2 mg bis 1 g/Tag Levallorphan.

11. Verwendung nach Anspruch 5, wobei die Zusammensetzung zur Anwendung in Mengen vorliegt, die folgende Dosen ergeben:

250 mg/Woche bis 2500 mg/Tag Chinolin-Derivat
100 bis 2500 mg/Tag Chinacrin
100 mg bis 10 g/Tag Chinin
100 mg bis 2 g/Tag Chinidin oder
100 mg bis 10 g/Tag Procain
10 bis 100 mg/Tag Emetin oder
100 mg bis 10 g/Tag Metronidazol oder
30 mg bis 2 g/Tag Spironolacton.

12. Verwendung nach einem der vorstegehenden Ansprüche, wobei die γ-Linolensäure vorliegt als Öl des Samens von Oenothera biennis, O. Lamarckiana oder einer anderen Nachtkerzenart oder eines Bestandteils desselben.

**Revendications pour Etats Contractants: CH DE FR GB IT LI LU NL SE BE**

1. Une composition pharmaceutique comprenant de l'acide γ-linolénique et/ou de l'acide dihomo-γ-linolénique ou un de leurs sels esters ou autres dérivés fonctionnels physiologiquement acceptables et de la thioproline, seuls ou dans un véhicule acceptable en pharmacie.

2. Une composition selon la revendication 1, comprenant en outre un composé de zinc physiologiquement assimilable, un antibiotique de type β-lactame, de la pénicillamine, de la phenformine ou du levamisole.

3. Une composition selon la revendication 1, comprenant en outre de la colchicine; ou de la vinblastine; ou de la griséofulvine, de l'amantadine ou de la mélatonine; ou de l'interféron.

4. Une composition selon la revendication 1, comprenant en outre de la vitamine C; ou de la naloxone, de la nalorphine ou du lévallorphan.

5. Une composition selon la revendication 1, comprenant en outre un dérivé de quinoléine choisi parmi la chloroquine, l'amodiaquine, l'hydroxychloroquine, la diiodohydroxyquine, l'iodochlorohydroxyqine, le chiniofon, la pentaquine, l'isopentaquine ou la primaquine; la quinacrine (mépacrine), la quinididine, la quinine ou la procaïne; l'émétine ou le métronidazole; ou la spironolactone.

6. Une composition selon l'une quelconque des revendications précédentes, présentée pour l'administration en quantités donnant 0,05 à 10 g par jour dudit acide γ-linolénique ou dihomo-γ-linolénique ou de son dérivé, calculé en acide γ-linolénique.

7. Une composition selon l'une quelconque des revendications précédentes, présentée pour l'administration en quantités donnant 100 mg à 35 g par jour de thioproline.

8. Une composition selon la revendication 2, présentée pour l'administration en quantités donnant 2,5 à 800 mg par jour d'un compose de zinc assimilable, calculé en zinc, 0,05 à 10 g par jour d'un antibiotique du type β-lactame, 50 mg à 10 g par jour de pénicillamine, 10 mg à 5 g par jour de phenformine ou 10 mg à 2 g par jour de lé amisole.

9. Une composition selon la revendication 3, présentée pour l'administration en quantitiés donnant des doses de:

0,3 à 15 mg par jour de colchicine,
100 à 1000 mg par jour d'amantadine,
0,5 à 5 g par jour de griséofulvine,
35 à 350 mg par semaine de vinblastine,
$1 \times 10^5$ à $1 \times 10^8$ unités par jour d'interféron ou new line 10 mg à 5 g par jour de mélatonine.

10. Une composition selon la revendication 4, présentée pour l'administration en quantités donnant des doses de:

50 mg à 50 g par jour d'acide ascorbique ou
0,1 à 500 mg par jour de naloxone,
1 mg à 5 g par jour de nalorphine ou
0,2 mg à 1 g par jour de lévallorphan.

11. Une composition selon la revendication 5, présentée pour l'administration en quantitiés donnant des doses de:

250 mg par semaine à 2500 mg par jour du dérivé de quinoléine,
100 à 2500 mg par jour de quinacrine,
100 mg à 10 g par jour de quinine,
100 mg à 2 g par jour de quinidine ou
100 mg à 10 g par jour de procaïne;
10 à 100 mg par jour d'émétine; ou
100 mg à 10 g par jour de métronidazole ou
30 mg à 2 g par jour de spironolactone.

12. Une composition selon l'une quelconque des revendications précédentes, dans laquelle l'acide γ-

linolénique est présent sous forme de l'huile de graines d'Oenethera biennis, d'O. Lamarckiana ou d'autres espèces d'oenothère ou de l'une de ses fractions.

13. Une composition selon l'une quelconque des revendications 1 à 11, dans laquelle l'acide γ-linolénique est présent sous la forme de l'huile de graines de Borago officinalis ou d'autres espèces de bourrache ou d'une de leurs fractions.

14. L'utilisation de l'acide γ-linolénique et/ou de l'acide dihomo-γ-linolénique ou d'un de leurs sels, esters ou autres dérivés fonctionnels physiologiquement acceptables et de thioproline dans la fabrication d'un produit pour le traitment conjoint du cancer.

15. Un conditionnement pharmaceutique comprenant les substances indiquées dans l'une quelconque des revendications précedentes comprenant au moins les deux composés essentiels indiqués dans la revendication 1 présentés séparément, ou bien un ou plus séparément et les autres ensemble, mais pour l'administration conjointe.

**Revendications pour Etat Contractant: AT**

1. L'utilisation de l'acide γ-linolénique et/ou de l'acide dihomo-γ-linolénique ou un de leurs sels, esters ou autres dérivés fonctionnels physiologiquement acceptables, conjointement avec la thioproline, seuls ou dans un véhicule acceptable en pharmacie pour le procédé de production d'une composition pharmaceutiqie pour le traitment du cancer.

2. L'utilisation selon la revendication 1, la composition comprenant en outre un composé de zinc physiologiquement assimilable, un antibiotique du type β-lactame, de la pénicillamine, de la phenformine ou du lévamisole.

3. L'utilisation selon la revendication 1, la composition comprenant en outre de la colchicine; ou de la vinblastine; ou de la griséofulvine, de l'amantadine ou de la mélatonine; ou de l'interféron.

4. L'utilisation selon la revendication 1, la composition comprenant en outre de la vitamine C; ou de la naloxone, de la nalorphine ou du lévallorphan.

5. L'utilisation selon la revendication 1, la composition comprenant en outre un dérivé de quinoléine choisi parmi la chloroquine, l'amodiaquine, l'hydroxychloroquine, la diiodohydroxyquine, l'iodochloro-hydroxyquine, le chiniofon, la pentaquine, l'isopentaquine ou la primaquine; la quinacrine (mépacrine), la quinidine, la quinine ou la procaïne; l'émétine ou le métronidazole; ou la spironolactone.

6. L'utilisation selon l'une quelconque des revendications précédentes, la composition étant présentée pour l'administration en quantités donnant 0,05 à 10 g par jour dudit acide γ-linolénique ou dihomo-γ-linolénique ou de son dérivé, calculé en acide γ-linolénique.

7. L'utilisation selon l'une quelconque des revendications précédentes, la composition étant présentée pour l'administration en quantités donnant 100 mg à 35 g par jour de thioproline.

8. L'utilisation selon la revendication 2, la composition étant présentée pour l'administration en quantités donnant 2,5 à 800 mg par jour d'un composé de zinc assimilable, calculé en zinc, 0,05 à 10 g par jour d'un antibiotique du type β-lactame, 50 mg à 10 g par jour de pénicillamine, 10 mg à 5 g par jour de phenformine ou 10 mg à 2 g par jour de lé amisole.

9. L'utilisation selon la revendication 3, la composition étant présentée pour l'administration en quantitiés donnant des doses de:

0,3 à 15 mg par jour de colchicine,
100 à 1000 mg par jour d'amantadine,
0,5 à 5 g par jour de griséofulvine,
35 à 350 mg par semaine de vinblastine,
$1 \times 10^5$g à $1 \times 10^8$ unités par jour d'interféron ou 10 mg à 5 g par jour de mélatonine.

10. L'utilisation selon la revendication 4, la composition étant présentée pour l'administration en quantités donnant des doses de:

50 mg à 50 g par jour d'acide ascorbique ou
0,1 à 500 mg par jour de naloxone,
1 mg à 5 g par jour de nalorphine ou
0,2 mg à 1 g par jour de lévallorphan.

11. L'utilisation selon la revendication 5, la composition étant présentée pour l'administration en quantitiés donnant des doses de:

250 mg par semaine à 2 500 mg par jour du dérivé de quinoléine,
100 à 2 500 mg par jour de quinacrine,
100 mg à 10 g par jour de quinine,
100 mg à 2 g par jour de quinidine, ou
100 mg à 10 g par jour de procaïne;
100 à 100 mg par jour d'émétine; ou
100 mg à 10 g par jour de métronidazole; ou
30 mg à 2 g par jour de spironolactone.

12. L'utilisation selon l'une quelconque des revendications précédentes, l'acide γ-linolénique étant présent sous forme de l'huile de graines d'Oenethera biennis, d'O. Lamarckiana ou d'autres espèces d'oenothère ou de l'une de ses fractions.